## Europäisches Patentamt

## European Patent Office

## Office européen des brevets

(19)

(11) Veröffentlichungsnummer: **0 137 505**
**B1**

(12)

# EUROPÄISCHE PATENTSCHRIFT

(45) Veröffentlichungstag der Patentschrift:
16.09.87

(21) Anmeldenummer: **84112228.6**

(22) Anmeldetag: **11.10.84**

(51) Int. Cl.⁴: **C 07 D 251/28**

(54) **Verfahren zur Gewinnung von Cyanurchlorid.**

(30) Priorität: **11.10.83 DE 3336994**

(43) Veröffentlichungstag der Anmeldung:
**17.04.85 Patentblatt 85/16**

(45) Bekanntmachung des Hinweises auf die Patenterteilung:
**16.09.87 Patentblatt 87/38**

(84) Benannte Vertragsstaaten:
**BE CH DE FR GB IT LI NL**

(56) Entgegenhaltungen:
**AT - B - 349 477**
**DE - B - 2 106 675**

**Die Akte enthält technische Angaben, die nach dem Eingang der Anmeldung eingereicht wurden und die nicht in dieser Patentschrift enthalten sind.**

(73) Patentinhaber: **SKW Trostberg Aktiengesellschaft, Dr.-Albert-Frank-Strasse 32 Postfach 1150/1160, . D-8223 Trostberg (DE)**

(72) Erfinder: **Vollbrecht, Heinz-Rüdiger, Dr., Herzog-Nikolaus-Strasse 15, D-8221 Stein/Traun (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

EP 0 137 505 B1

## Beschreibung

Gegenstand der vorliegenden Erfindung ist ein Verfahren zur Gewinnung von Cyanurchlorid in fester oder flüssiger Form aus verunreinigtem Cyanurchloriddampf.

Cyanurchlorid stellt ein wichtiges technisches Zwischenprodukt bei der Herstellung von Pflanzenschutzmitteln, Farbstoffen, optischen Aufhellern, Pharmazeutika sowie Textil- und Kautschukhilfsmitteln dar. Es fällt nach der Trimerisierung von Chlorcyan mit Hilfe von Katalysatoren in gasförmiger Form an, wobei es im wesentlichen als Gemisch mit überschüssigem Chlorcyan und Chlor vorliegt. Dieses Gasgemisch wurde üblicherweise in Abscheidekammern geleitet, wobei es an den gekühlten Wandflächen in die feste Form übergeführt wurde. Nachteilig bei dieser Desublimation war, dass sich das Cyanurchlorid in Form grober Kristalle an den Wänden und Austragsvorrichtungen absetzte, was zu erheblichen technischen Schwierigkeiten führte, ganz abgesehen von der schlechten Qualität des dabei erhaltenen Produktes. Zur Beseitigung dieses Problems wurde gemäss DE-AS 1266308 versucht, dass Cyanurchlorid zusammen mit einer leicht verdampfenden inerten Kühlflüssigkeit wie z. B. Chloroform mittels einer Zerstäuberdüse zu versprühen. Man erhält auf diese Weise zwar feinverteiltes Cyanurchlorid, doch kommt es bei diesem Verfahren sehr leicht zu Verstopfungen der Düse. Darüber hinaus ist die Rückgewinnung des Kühlmediums technisch nicht einfach.

Wegen der schwierigen Handhabung des festen Cyanurchlorids ist es in den Fällen, in denen eine schnelle Weiterverarbeitung des Cyanurchlorids erfolgt, wünschenswert, das Cyanurchlorid in flüssiger Form zu gewinnen.

Aus der DE-PS 2332636 ist bekannt, gasförmiges Cyanurchlorid zusammen mit einer Hilfsflüssigkeit in einer Fraktionierkolonne zu kondensieren; nachteilig ist dabei aber der grosse technische Aufwand zur Aufarbeitung der Hilfsflüssigkeit sowie zur Reinigung der Abgase.

In der DE-AS 2843381 und in der DE-AS 2843382 werden Verfahren beschrieben, nach denen wahlweise flüssiges oder festes Cyanurchlorid hergestellt werden kann, welche dadurch gekennzeichnet sind, dass man das gasförmige Reaktionsgemisch in eine Apparatekombination bestehend aus Abtriebskolonne und Kondensator einleitet und durch Temperaturregelung am Austritt des Kondensators das Cyanurchlorid in der Kolonne teilweise kondensiert, während das gasförmige Cyanurchlorid, welches am Kolonnenkopf austritt, in den üblichen Abscheidekammern als Feststoff anfällt. Auch diese Verfahren erfordern wegen der aufwendigen Apparatekombination hohe Investitions- und Betriebskosten und stellen somit keine optimale Lösung dieses Problems dar.

Schliesslich wird in der AT-A-349477 ein Verfahren zur Herstellung von feinteiligem, festem Cyanurchlorid beschrieben, bei dem flüssiges Cyanurchlorid in einer ersten Abscheidekolonne mit einem inerten Gas gekühlt wird, um es in kristalliner Form auszuscheiden und das dabei entstehende cyanurchloridhaltige Gas in einer zweiten Abscheidekolonne mit einer Cyanurchlorid gut lösenden Hilfsflüssigkeit vom verdampften Cyanurchlorid freigewaschen wird. Sowohl Gas als auch Hilfsflüssigkeit werden durch ein aufwendiges und kompliziertes Verfahren recycliert.

Der vorliegenden Erfindung lag daher die Aufgabe zugrunde, ein Verfahren zur Gewinnung von Cyanurchlorid in fester und/oder flüssiger Form zu entwickeln, welches die genannten Nachteile nicht aufweist und es mit technisch einfachen Mitteln ermöglicht, Cyanurchlorid wahlweise in flüssiger oder fester Form oder in beiden Formen zu gewinnen, und das auch hinsichtlich Reinheit und Kornverteilung des Feststoffes gute Ergebnisse liefert.

Diese Aufgabe wird erfindungsgemäss durch das im Anspruch 1 definierte Verfahren gelöst. Die Unteransprüche betreffen bevorzugte Ausführungen des erfindungsgemässen Verfahrens. Es hat sich nämlich überraschenderweise gezeigt, dass man auch ohne Zuhilfenahme einer Kolonne wahlweise flüsssiges oder festes Cyanurchlorid mit hoher Reinheit gewinnen kann, wobei sich das feste Cyanurchlorid darüber hinaus durch ein besonderes feinteiliges Kornspektrum auszeichnet. Es war auch nicht zu erwarten, dass sich dieses Verfahren technisch problemlos, z. B. ohne Verstopfungen, durchführen lässt.

Beim erfindungsgemässen Verfahren wird der Cyanurchloriddampf, der noch zahlreiche Verunreinigungen, wie z. B. Chlorcyan oder Chlor enthalten kann, mit einem bei einer Temperatur von 60–140 °C, insbesondere 100–120 °C, befindlichen Luftstrom verdünnt und anschliessend in die üblichen Abscheidekammern eingeleitet, wobei die Luftstrommenge zwischen 250 und 750, insbesondere 400–500 l/kg gasförmigem Cyanurchlorid beiträgt. Durch die erfindungsgemässe Verdünnung des Cyanurchloriddampfes kann erreicht werden, dass das Cyanurchlorid in den Leitungen nicht kondensiert und zu unerwünschten Verstopfungen führt.

Darüber hinaus wird beim Einleiten des Cyanurchloriddampfs in die Abscheidekammer durch Turbulenzbildung verhindert, dass sich bereits im oberen Teil der Abscheidekammer grobe Kristalle an den Wänden absetzen. Die Desublimation des festen Cyanurchlorids erfolgt vielmehr im unteren Teil der Abscheidekammer, die eine Kühltemperatur von 20–50 °C, insbesondere 30–40 °C, aufweisen soll. Auf diese Weise kann schon eine gute Reinigung und eine ausgezeichnete Kornverteilung des festen Produkts erzielt werden. Die Abgase werden zusammen mit dem Luftstrom oberhalb der Desublimationszone abgezogen, das feste Cyanurchlorid wird ausgetragen und je nach Bedarf ganz oder teilweise in einen oberhalb 146 °C befindlichen Schmelzbehälter eingeleitet, um das Cyanurchlorid in die flüssige Form überzuführen. Der Kreislauf wird dadurch geschlossen, dass man das bei der entsprechenden Schmelztemperatur gasförmige Cyanurchlorid, welches noch Spuren an Verunreinigungen enthalten kann, zusammen mit dem 60–140°C heissen Luftstrom in die Abscheidekammer zurückführt. Die Wahl der Schmelztemperatur hat einen wesentlichen Einfluss auf die Reinheit des Cyanurchlorids, denn je höher diese Temperatur eingestellt wird, desto mehr gasförmiges Cyanurchlorid wird im Kreislauf geführt und desto besser ist die Reinigungswirkung, weil die Abgase ja ständig aus der Abscheidekammer abgezogen werden. Das erfindungsgemässe Verfahren ermöglicht nicht nur die wahlweise Gewinnung von festem oder flüssigem Cyanurchlorid, sondern stellt auch zugleich ein Reinigungsverfahren dar, wobei nicht unbedingt von gasförmigem Cyanurchlorid ausgegangen werden muss. In einer Ausführungsform kann auch festes Cyanurchlorid in die Schmelze eingebracht werden. Das verdampfte Cyanurchlorid kann solange im Kreislauf geführt werden, bis es die erforderliche Reinheit aufweist.

Zur Aufrechterhaltung der Schmelztemperatur ist es energetisch besonders günstig, die Abwärme, die z.B. bei der Trimerisierung des Chlorcyans und/oder der Abscheidung des Cyanurchlorids anfällt, auszunützen.

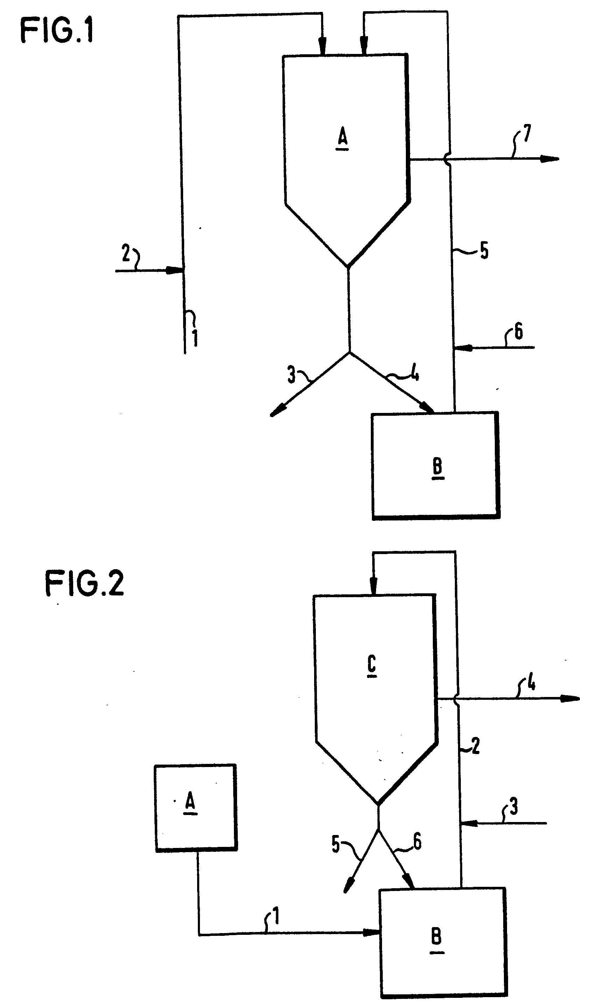

Das erfindungsgemässe Verfahren wird anhand der Abbildungen 1 und 2 und der Beispiele 1 und 2 näher erläutert.

Abbildung 1 zeigt schematisch die Gewinnung von festem oder flüsigem Cyanurchlorid aus Cyanurchloriddampf, wie er z.B. nach der Trimerisierung des Chlorcyans anfällt. Der Cyanurchloriddampf wird durch die Leitung 1 der Abscheidekammer A zugeführt, nachdem über Leitung 2 ein Luftstrom zudosiert worden ist. Nach der Desublimation wird der Feststoff über Leitung 3 ausgetragen oder über 4 in den Schmelzbehälter B geführt. Die Rückführung des Cyanurchloriddampfes aus dem Schmelzbehälter in die Abscheidekammer erfolgt nach der Verdünnung mit der aus 6 zugeführten Luft über Leitung 5, während die Abgase aus der Abscheidekammer über 7 abgezogen werden.

Abbildung 2 zeigt das Blockschema für die Reinigung von festem Cyanurchlorid mit Hilfe des erfindungsgemässen Verfahrens. Aus dem Vorratsbehälter A wird festes Cyanurchlorid über die Zuleitung 1 in den Schmelzbehälter B geführt, der durch Leitung 2 mit der Abscheidekammer C verbunden ist. Durch 2 gelangt der Cyanurchloriddampf nach Verdünnung mit dem Luftstrom aus 3 in C und wird dort als Feststoff abgeschieden, während die Abgase über 4 die Abscheidekammer verlassen. Der Austrag des festen Cyanurchlorids erfolgt über Leitung 5 und die Rückführung des Feststoffs in die Schmelze über Leitung 6.

Beispiel 1

Es werden 200 kg Cyanurchloriddampf mit einer Temperatur von 170°C nach dessen Verdünnung mit 90 kg 100°C heisser Luft pro Stunde in die Abscheidekammer eingeleitet, deren unterer Teil eine Temperatur von 30°C aufweist. Nach der Desublimation werden 150 kg festes Cyanurchlorid ausgetragen und die restlichen 50 kg in die Schmelze eingebracht und dort kontinuierlich abgezogen. Das entsprechend dem Dampfdruck des Cyanurchlorids bei der Schmelztemperatur anfallende Cyanurchloridgas wird mit 20 kg Luft verdünnt und wieder in die Abscheidekammer zurückgeführt.

Die Analyse des auf diese Weise gewonnenen Produkts weist folgende Zusammensetzung auf:

| Cyanurchlorid | > 99,5% |
| Polymertriazine | < 0,1% |
| Hydroxytriazine | < 0,5% |

Das Kornspektrum des festen Cyanurchlorids hat folgende Zusammensetzung:

| > 250 µm | 0,8% |
| 125–250 µm | 2,5% |
| 63–125 µm | 2,3% |
| 40– 63 µm | 13,9% |
| < 40 µm | 80,5% |

Beispiel 2

100 kg festes Cyanurchlorid mit der Zusammensetzung:

| Cyanurchlorid | 98,53% |
| Polymertriazine | 0,57% |
| Hydroxytriazine | 0,90% |

werden aus einem Vorratsbehälter in den Schmelzbehälter eingebracht, der bei einer Temperatur von 190°C betrieben wird und der mit der Abscheidekammer verbunden ist. Der Cyanurchloriddampf wird vor Eintritt in die Abscheidekammer mit 20 kg Luft verdünnt, die eine Temperatur von 100°C aufweist. Das Produkt wird kontinuierlich abgeschieden und ausgetragen, wobei es folgende Analyse aufweist:

| Cyanurchlorid | > 99,5% |
| Polymertriazine | < 0,1% |
| Hydroxytriazine | < 0,5% |

Das Kornspektrum entspricht dem des Beispiels 1.

**Patentansprüche**

1. Verfahren zur Gewinnung von Cyanurchlorid in fester und/oder flüssiger Form aus einem verunreinigten Cyanurchloriddampf unter Verwendung von Luft als Hilfsmedium, dadurch gekennzeichnet, dass man den Cyanurchloriddampf mit einem 60–140°C heissen Luftstrom in einer Menge von 250–750 l Luft/kg Cyanurchlorid verdünnt und danach in eine im unteren Teil auf 20–50°C gekühlte Abscheidekammer einleitet, gegebenenfalls das abgeschiedene feste Cyanurchlorid ganz oder teilweise in einem oberhalb 146°C befindlichen Schmelzbehälter in die flüssige Form überführt und den Cyanurchloriddampf nach erneuter Verdünnung mit heisser Luft von 60–140°C aus dem Schmelzbehälter in die Abscheidekammer zurückführt, während man die cyanurchloridfreien Abgase oberhalb der Desublimationszone aus der Abscheidekammer abzieht.

2. Verfahren nach Anspruch 1, dadurch gekennzeichnet, dass die Temperatur des heissen Luftstroms 100–120°C beträgt.

3. Verfahren nach Anspruch 1 oder 2, dadurch gekennzeichnet, dass der untere Teil der Abscheidekammer eine Temperatur von 30–40°C besitzt.

4. Verfahren nach einem der vorhergehenden Ansprüche, dadurch gekennzeichnet, dass man dem Cyanurchloriddampf pro kg Cyanurchlorid heisse Luft in Mengen von 400–500 l zusetzt.

5. Verfahren nach einem der vorhergehenden Ansprüche, dadurch gekennzeichnet, dass man der im Schmelzbehälter befindlichen Cyanurchloridschmelze zusätzlich oder anstelle des der Abschiedekammer zugeführten Cyanurchloriddampfs gegebenenfalls verunreinigtes festes Cyanurchlorid zuführt.

6. Verfahren nach einem der vorhergehenden Ansprüche, dadurch gekennzeichnet, dass man den Cyanurchloriddampf solange im Kreislauf führt, bis der gewünschte Reinheitsgrad erreicht ist.

7. Verfahren nach einem der vorhergehenden Ansprüche, dadurch gekennzeichnet, dass zur Aufrechterhaltung der Schmelztemperatur die bei der Trimerisierung des Chlorcyans und/oder bei der Desublimation des Cyanurchlorids anfallende Abwärme ausgenützt wird.

**Claims**

1. Process for the obtaining of cyanuric chloride in solid and/or liquid form from a contaminated cyanuric chloride vapour with the use of air as adjuvant medium, characterised in that one dilutes cyanuric chloride with a hot air stream of 60–140°C in an amount of 250–750 l air/kg cyanuric chloride and thereafter introduces into the lower part of a separation chamber cooled to 20–50°C, optionally converts the separated solid cyanuric chloride wholly or partly into the liquid form in a melt container above 146°C and returns the cyanuric chloride vapour, after again diluting with hot air of 60–140°C, from the melt container into the separation chamber, whereas one withdraws the cyanuric chloride-free waste gases from the separation chamber above the desublimation zone.

2. Process according to claim 1, characterised in that the temperature of the hot air stream amounts to 100–120°C.

3. Process according to claim 1 or 2, characterised in that the lower part of the separation chamber has a temperature of 30–40°C.

4. Process according to one of the preceding claims, characterised in that one adds hot air to the cyanuric chloride in amounts of 400–500 l per kg of cyanuric chloride.

5. Process according to one of the preceding claims, characterised in that to the cyanuric chloride melt present in the melt container one introduces optionally impure solid cyanuric chloride in addition to or instead of the cyanuric chloride vapour introduced into the separation chamber.

6. Process according to one of the preceding claims, characterised in that one passes the cyanuric chloride vapour in a cycle until the desired degree of purity is achieved.

7. Process according to one of the preceding claims, characterised in that, for the maintenance of the melt temperature, there is utilised the waste heat obtained in the case of the trimerisation of the cyanogen chloride and/or in the case of the desublimation of the cyanuric chloride.

**Revendications**

1. Procédé de préparation du chlorure de cyanuryle sous forme solide et/ou liquide à partir d'une vapeur de chlorure de cyanuryle souillée d'impuretés, en utilisant l'air comme milieu auxiliaire, caractérisé en ce qu'on dilue la vapeur de chlorure de cyanuryle avec un courant d'air chaud à 60–140°C à raison de 250–750 litres d'air/kg de chlorure de cyanuryle, puis on l'envoie dans une chambre de dépôt refroidie à la partie inférieure à 20–50°C, on amène le cas échéant sous la forme liquide le chlorure de cyanuryle solide qui s'est déposé, en tout ou partie, dans un récipient de fusion se trouvant au-dessus de 146°C, et on renvoie la vapeur de chlorure de cyanuryle, après une nouvelle dilution avec de l'air chaud à 60–140°C du récipient de fusion dans la chambre de dépôt, tandis qu'on soutire de la chambre de dépôt les gaz résiduaires exempts de chlorure de cyanuryle au-dessus de la zone de désublimation.

2. Procédé suivant la revendication 1, caractérisé en ce que la température du courant d'air chaud est de 100–120°C.

3. Procédé suivant les revendications 1 ou 2, caractérisé en ce que la partie inférieure de la chambre de dépôt possède une température de 30 à 40°C.

4. Procédé suivant l'une des revendications précédentes, caractérisé en ce qu'on ajoute à la vapeur de chlorure de cyanuryle de l'air chaud à raison de 400–500 litres par kg de cyanure de cyanuryle.

5. Procédé suivant l'une des revendications précédentes, caractérisé en ce qu'on envoie dans la masse fondue de chlorure de cyanuryle se trouvant dans le récipient de fusion, en plus ou à la place de la vapeur de chlorure de cyanuryle envoyée à la chambre de dépôt, du chlorure de cyanuryle solide, le cas échéant souillé d'impuretés.

6. Procédé suivant l'une des revendications précédentes, caractérisé en ce qu'on envoie la vapeur de chlorure de cyanuryle dans le cycle jusqu'à ce qu'on ait atteint le degré de pureté désiré.

7. Procédé suivant l'une des revendications précédentes, caractérisé en ce que, pour le maintien de la température de fusion, on met à profit la chaleur perdue lors de la trimérisation du chlorure de cyanogène et/ou lors de la désublimation du chlorure de cyanuryle.

FIG.1

FIG.2